# EUROPEAN PATENT SPECIFICATION

(11) **EP 1 837 047 B1**
(45) Date of publication and mention of the grant of the patent: **27.02.2013**
(21) Application number: 07005925.8
(22) Date of filing: 22.03.2007
(51) Int. Cl.: A61M 5/14, A61M 5/44

(54) **Supporting device for containers of biomedical fluids for parenteral administering, particularly bottles and phials for medical fluids, blood or the like**
Haltevorrichtung für Behälter für biomedizinische Flüssigkeiten zur parenteralen Verabreichung, insbesondere Flaschen und Ampullen für medizinische Flüssigkeiten, Blut und dergleichen
Dispositif de support pour conteneurs de fluides biomédicaux pour une administration parentérale, spécialement les bouteilles et fioles pour fluides médicaux, ou fluides sanguins similaires

(30) Priority: 24.03.2006 IT MO20060098
(43) Date of publication of application: 26.09.2007
(73) Proprietor: SIDAM S.r.l., 41037 Mirandole-Franzione San Giacomo Roncole(MO) (IT)
(72) Inventor: Azzolini, Graziano, 41032 Cavezzo (MO) (IT)
(74) Representative: Brunacci, Marco

(56) References cited:
- WO-A-2005/056089
- DE-A1- 4 328 321
- FR-A- 1 146 377
- US-A- 1 457 796
- US-A- 4 804 367

## Description

The present invention refers to a supporting device for containers of biomedical fluids for parenteral administering, particularly bottles and phials for medical fluids, blood or the like.

It is a known fact that people with health problems, struck by indispositions and accident victims require medical care and attention that sometimes calls for the parenteral administering of biomedical fluids and/or liquids (drugs, physiological salt solutions, blood or the like) at a pre-established temperature.

In some cases for example, patients remain in a state of shock characterised by hypovolemia (volumetric reduction of the blood) and hypothermia (drop in body heat), and fluids have to be infused at a temperature generally between 33°C and 40°C.

For the treatment of tumoral illnesses, on the other hand, the need often arises to administer chemotherapy products the effectiveness of which varies according to temperature and which, therefore, have to be infused at a temperature generally above room temperature.

Similar considerations can be made as regards the means of contrast used in diagnostics, these too commonly administered at the patient's body temperature. In conditions of brain distress, on the other hand, it is often best to reduce the body temperature of the patient by means of the infusion of cooled fluids.

The fluids to be injected are currently packaged in containers, of the flexible bag, plastic phial or glass bottle type, that have a dispensing mouth from where the fluid can be taken by means of a standard syringe or by means of a sampling and transport duct, commonly called a "defluxion device", the exit end part of which is associated with a patient injection element (catheter or needle).

To condition the temperature of the fluids, use is normally made of particular systems for heating and/or cooling the fluid containers and/or the fluid sampling and transport duct.

To cool the fluid to be infused, for example, use is usually made of iced water containers, in which the sampling and transport duct is manually immerged by a health operator every time the need is felt; in such cases however, the control of the temperature of the fluid at the time of the infusion is absolutely subjective and uncertain and risks jeopardising the effectiveness of the treatment and/or the health of the patient unless performed by expert and professional medical staff.

To heat the fluid, on the other hand, the use is known of devices essentially made up of a box inside which the containers are placed connected to the patient by means of the defluxion devices and which, during the dispensing of the fluid, is heated by heat production means, e.g. of the electric resistor type; such box also has an electronic temperature control and adjustment circuit.

Other devices of known type heat the sampling and transport duct during administering and essentially consist of a metal element which is coupled with electric resistors, supplied with electricity, and which is placed close to the portion of the defluxion device to be conditioned; in this case as well an electronic circuit controls and adjusts the achieved temperature.

These devices for heating the container and/or the sampling and transport duct have a number of drawbacks among which we can recall the fact that they are of considerable weight and overall dimensions that makes transport and use particularly difficult and laborious, that they are complex in terms of structure and construction, that they require the intervention of skilled operators and are fairly costly.

Another drawback of these known heating devices consists in the fact that, using electricity, they can create interferences with other electric/electronic devices located near the patient.

Devices are also known for heating or cooling the fluid containers which, in jargon, are defined as being of the so-called "chemical" type because they exploit the heat produced by an exothermic or endothermic chemical reaction between two or more reacting substances.

These devices of "chemical" type are essentially composed of a wrapping, of the bag type, split into two or more compartments separated the one from the other by a tearable membrane and of which one contains a first component and the other contains a second component suitable for reacting together with an exothermic or endothermic reaction depending on the type of intended use of such devices.

At the time of use, an operator acts manually on the wrapping, exercising on this a pressure such as to break the tearable membrane and allow the chemical combination of the components, and then manually places the device in contact with the container of the fluid to be administered to thermally condition it.

These "chemical" type devices also have a number of drawbacks such as, for example, the fact that they have to be stored, transported and handled with care and attention to prevent suffering accidental knocks that involuntarily and at unsuitable times activate the reaction between the chemical components they contain, with the risk of prematurely exhausting their capacity to develop calories/refrigeration units and, therefore, become unusable at the time of actual need.

Another drawback of the "chemical" type devices consists in the fact that they contain air which, because it has a low heat conduction, acts as insulation and reduces the exchange of heat with the containers of the fluids to be conditioned. As an alternative to the previously described systems and devices, the use is known of special heating appliances, or cooling appliances, composed of an electric oven, or a cool cell, inside which the containers are placed to keep the fluid at the desired temperature and from which they are removed only an instant before being used.

The use of such appliances, though very easy and functional, nevertheless permits administering the fluid at the required temperature only for a limited period of time because, once taken out of the ovens, or of, the cool cells, the containers abruptly start to exchange heat with the outside and quickly tend to reach room temperature.

From patent document FR 1 146 377 it is known a device, for the realization of operations of transfusion and analogues, which comprises a cylindrical hollow support, made of an insulating material, in which a bottle or flacon containing the liquid to be transfused to a patient can be housed.

An additional ice pack can be arranged at the inside of the insulating cylindrical support for cooperating with it in order to maintain the liquid temperature constant in the time.

It is also known from DE 43 28 321 A1 a device for controlling the temperature of infusion solutions which comprises a cup-shaped vessel suitable for receiving and supporting infusion bottles or bags, wherein this cup-shaped vessel can be heated during infusion by suitable electrical heating means.

However the solutions disclosed by these patent documents appear to require further improvements, in particular in the sense of allowing a more practical handling and/or avoiding the use of specific electrical heating means for holding at the desired temperature in the time the medical fluid to be administered to a patient.

The main aim of the present invention is to provide a supporting device for containers of biomedical fluid for parenteral administering, particularly bottles and phials for medical fluids, blood or the like, that favours the infusion to patients of fluids brought to a pre-established temperature without requiring, during administering, complicated and cumbersome heating/cooling systems/devices.

A further object of the present invention is to provide a device that can be used in conditions of utmost safety for the patient and maximum efficiency of use.

Another object of the present invention is that it is simple in terms of structure and construction, has limited weight and overall dimensions and is easy to transport and handle including by inexpert health operators.

The above-indicated objects are all reached by the present supporting device for containers of biomedical fluids for parenteral administering, particularly bottles and phials for medical fluids, blood or the like, comprising at least one element substantially shaped like a cup, having at least one upper fitting opening for fitting a container for biomedical fluids and at least one lower exit opening of the fluid, characterized in that said cup element comprises at least one outer surface made of heat insulating material and at least one inner surface suitable for facing said container and of which at least one portion is associable with heat energy conduction and accumulation means.

Further characteristics and advantages of this invention will appear even more evident from the detailed description of a preferred, but not exclusive, embodiment of a supporting device for containers of biomedical fluids for parenteral administering, particularly bottles and phials for medical fluids, blood or the like, illustrated indicatively by way of non limiting example, in the attached drawings wherein:
figure 1 is an axonometric view of a first embodiment of the device according to the invention, used for supporting a bottle containing medical fluids;
figure 2 is a schematic and partial section view of the device of figure 1;
figure 3 is an axonometric view of a second embodiment of the device according to the invention;
figure 4 is an axonometric view of a further embodiment of the device according to the invention;
figure 5 is a schematic and partial section view of the device of figure 4;
figure 6 is an axonometric view of the embodiment of figure 4 with two cup elements.

With particular reference to such figures, a supporting device has been globally indicated by 1 for containers of biomedical fluids for parenteral administering, particularly bottles and phials for medical fluids, blood or the like.

In the particular embodiments shown in the figures, the container R consists of a rigid bottle for containing the fluid L to be injected in a patient for diagnostic purpose according to the diagnostic techniques; such bottle has a neck C converging and terminating in a reclosable dispensing mouth B.

It cannot however be ruled out that the present invention can also be used with flexible bags, semi-rigid phials or other type of containers for biomedical use, containing blood, drugs or other substances to be administered.

The device 1 comprises a substantially rigid cup element 2 having, at the top, a fitting opening 3 for fitting the container R and, in the lower portion, a fluid exit opening 4.

The cup element 2 comprises an outer surface 5 made of heat insulating material, e.g. of the polymeric type, and an inner surface 6 which, during use, is suitable for being arranged facing the container R and which is associated with heat energy conduction and accumulation means 7.

Such conduction and accumulation means are composed of a first layer 8 made of heat conductive material, which defines the inner surface 6 of the cup element 2, and of a second layer 9 made of heat insulating material, which is suitable for accumulating heat energy (calories or refrigeration units) and substantially placed in between the outer surface 5 and the inner surface 6.

The heat conductive material of the first layer 8 is of the metal type, e.g. aluminium.

The outer surface 5 and the first layer 8 define between them an interspace insulated from the outside and completely filled with the heat insulating material of the second layer 9 which, advantageously, is in the fluid state (air or liquid).

The cup element 2 has a cross section substantially circular in shape and a lower portion 2a shaped like a funnel converging downwards, on which rests the neck C of the container R once this has been fitted through the fitting opening 3.

In use configuration, the dispensing mouth B of the container R protrudes from the exit opening 4.

The fitting opening 3 has substantially larger dimensions than the exit opening 4; the fitting opening 3, as a matter of fact, is defined by the upper open edge of the cup element 2, while the exit opening 4 is defined by the lower open edge of the funnel-shaped lower portion 2a.

The inner surface 6 of the cup element 2, in actual fact, is substantially complementary to the shape of the container R, so that, during use, the inner surface 6 of the cup element 2 and the outer surface of the container R are placed in reciprocal contact of the conforming type.

Alternative embodiments of the present invention cannot, however, be ruled out where the dimensions of the cup element 2 are substantially larger than the container R, in this case dimension adapting means can be usefully provided, suitable for transmitting heat between the cup element 2 and the container R without the interposition of air bags.

Such adapting means, not shown in detail in the figures, can consist, for instance, of an additional tubular shaped body, made of heat conductive material and having an outer side complementary to the inner surface 6 of the cup element 2 and which can be placed exactly in contact with this, and of an inner side complementary to the container R and which can be placed exactly in contact with this.

The heat conductive material of the additional body, when provided, can usefully coincide with the heat conductive material of the first layer 8 and be, for example, in aluminium.

Close to the fitting opening 3 are provided coupling means 10 of the cup element 2 to a supporting hook G, e.g. of the type of those at the top of the traditional drip support rods A commonly used in hospitals and medical clinics.

In detail, the coupling means 10 are defined by a brace which is associated with diametrically opposite parts of the outer surface 5 of the cup element 2 and can be hung on the supporting hook G.

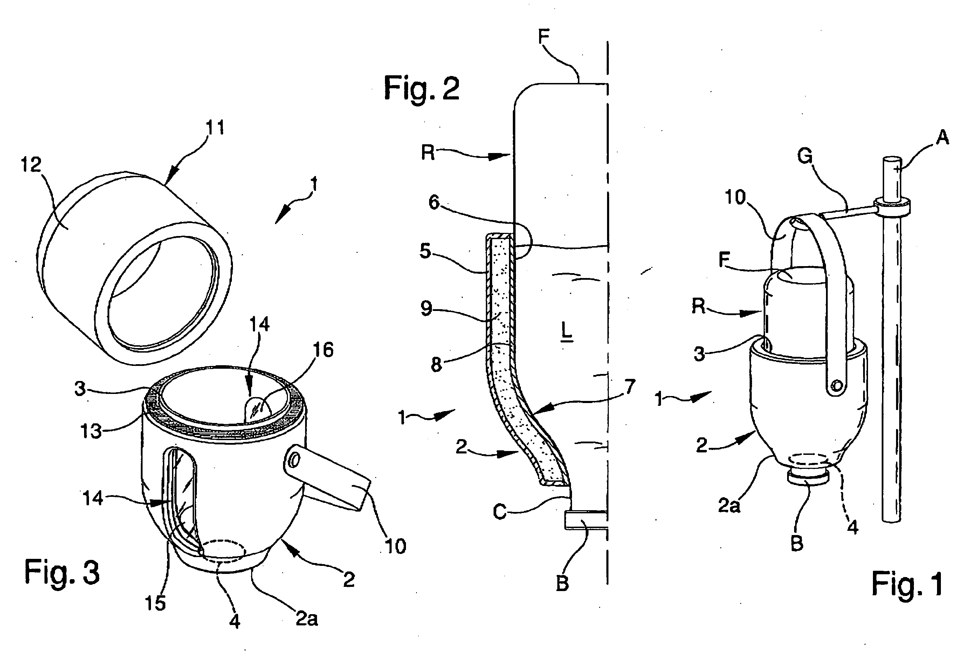

Figures 1 and 2 show a first embodiment of the invention wherein the height of the cup element 2 is such that, in use configuration, the bottom F of the container R remains outside the element itself.

In consideration of the fact that, in use configuration, the fluid L to be administered collects near the dispensing mouth B and the neck C of the container R, it can easily be understood how the cup element 2 is able to exchange heat energy with the part of the container R directly in contact with the fluid L, allowing this to be heated/cooled in an efficient way.

In a second embodiment of the invention shown in figure 3, on the other hand, the device 1 is fitted with closing means 11 for closing the fitting opening 3 which are of the removable type, i.e. they can be fitted once the container R has been introduced into the cup element 2 and removed when the container R needs to be pulled out.

In detail the closing means 11 consist of a cover 12 shaped so as to define a semi-shell which, in operating configuration, is suitable for covering the portion of the container R protruding from the opening itself, so as to completely heat insulate the container R from the external environment.

The open edge of such cover 12, in detail, is associable with the edge of the fitting opening 3 by interposition of sealing means 13, of the type of a ring-shaped seal fixed to the edge of the fitting opening 3; it should be noted how, in closing configuration, such sealing means prevent the passage of air, and therefore heat, between the external environment and the inside of the device 1.

Unlike the embodiment shown in figures 1 and 2, in the second embodiment the device 1 is fitted with sight inspection means 14 for inspecting the level of the fluid L inside the container R.

Such sight inspection means comprise a transparent window 15, elongated vertically, which is obtained through a side of the cup element 2.

In consideration of the fact that most fluids used in the biomedical field are transparent and colourless, in order to increase the visibility through the window 15, the sight inspection means 14 also comprise a reflecting body 16, of the mirror type or the like, associated with the inner surface 6 of the cup element 2 at the diametrically opposite part with respect to the window 15.

The present invention according to the embodiments of figures from 1 to 3 operates as follows.

The container R and the device 1 are initially heated, or cooled, by means of a known conditioning apparatus, e.g. inside an electric oven, a cool cell or the like.

During this initial phase of heat conditioning, the container R and the device 1 are left separate the one from the other, allowing the container R to reach the temperature required for administering to the patient and the cup element 2 to accumulate calories, or refrigeration units, inside the first and second layer 8 and 9.

Afterwards the cup element 2 is removed from the electric oven/cool cell together with the container R, is hung to the supporting hook G and the container R is fitted inside through the fitting opening 3.

When provided, the cover 12 can therefore be fitted to the cup element 2 to completely insulate the container R from the external environment.

During administering to patient, the quantity of biomedical fluid L which remains inside the container R tends to exchange heat with the external environment but with a very limited temperature gradient since the heat energy initially accumulated by the first and second layer 8 and 9 is progressively transferred to the container R which maintains the temperature required for administering to the patient for a long time.

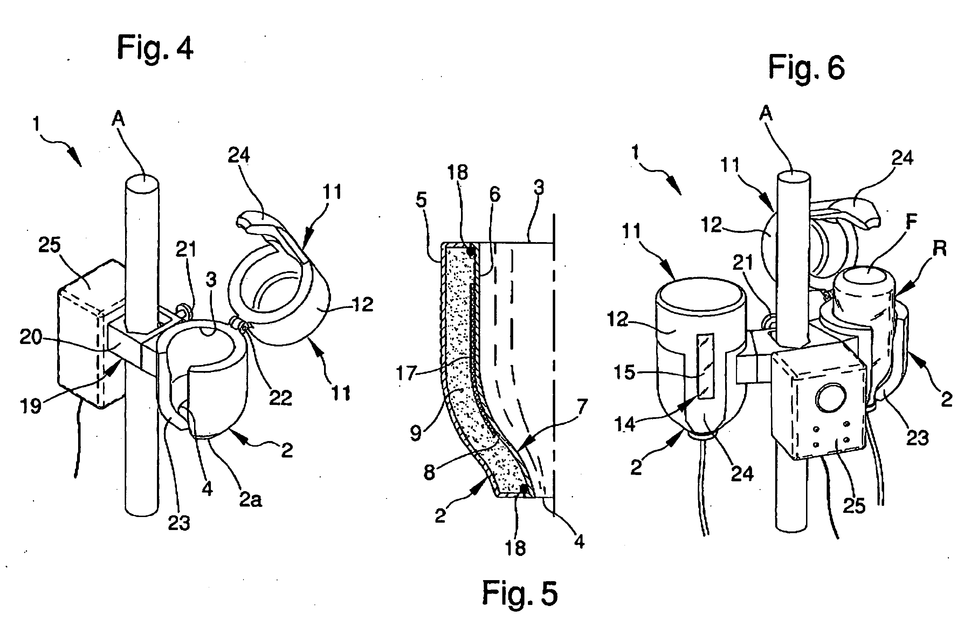

In the figures from 4 to 6 a further embodiment of the invention is shown, wherein, conveniently, heating means 17 are arranged inside a cup element 2 (figure 4) or two cup elements 2 (figure 6).

The heating means 17 consist, e.g., of a electric resistor arranged in contact with the inner side of the first layer 8.

At the axial end parts of the first layer 8, on the joining areas between the first layer and the outer surface 5, are arranged packing means 18, of the type of a pair of rubber O-rings, suitable for preventing any fluid from flowing inside the cup element 2 and, in particular, the contrast liquid L in case of accidental breaking of the container R.

The heating means 17, in actual fact, allow heating the first layer 8 without having to place the cup element or cup elements 2 inside an electric oven or the like.

This feature allows fitting the device 1 with gripping means 19 associated with the cup element 2 and suitable for allowing their mounting, in a firm and rigid way, on the vertical upright of the drip support rod A.

The gripping means 19 are of the clamp type and comprise a block 20, associated integral with the cup element 2 and which can be positioned around the drip support rod A, and screw means 21 which are engaged through the block 20 and suitable for pressing against the rod A.

In the embodiment of the figures from 4 to 6, the device I has a cover 12 for each cup element 2 but, unlike the embodiment of figure 3, hinging means 22 are provided for hinging the cover itself to the edge of the fitting opening 3.

Furthermore, each cup element 2 has a longitudinal slot 23 which extends from one side to the other between the fitting opening 3 and the exit opening 4.

In the closing configuration, the space defined by the longitudinal slot 23 is intended to be filled by an elongated complementary wall 24, which extends from the cover 12 and on which the sight inspection window 15 is obtained.

Usefully, the operation of the heating means 17 is controlled by means of a central processing and control unit 25 which is associated with the cup element/s 2 by interposition of the block 20.

The present invention according to the embodiment of the figures from 4 to 6 operates as follows.

The central processing and control unit 25, in actual fact, allows controlling the temperature of the first layer 8 and interrupting the power supply to the electric resistor 17 once verified that a pre-established temperature has been reached.

When the first and second layers 8 and 9 have accumulated enough heat, the cup element/s 2 is/are able to maintain the temperature of the biomedical fluid L inside the container R for a long time.

Furthermore, in the particular case of the device 1 having two cup elements 2 (figure 6), the central processing and control unit 25 is able to control the alternate heating of the cup elements 2.

By so doing, while one cup element 2 is in the heating phase by means of its own heating element, the other cup element 2 has already accumulated enough heat and is used for administering the biomedical fluid L to the patient.

This makes it possible to always have at disposal at least one cup element 2 the conduction and accumulation means 7 of which are warm and ready for use.

It has in fact been found that the described invention achieves the proposed objects.

The invention thus conceived is susceptible of numerous modifications and variations, all of which falling within the scope of the inventive concept

Furthermore, all the details may be replaced by other elements which are technically equivalent.

In practice, the materials used, as well as the contingent shapes and dimensions, may be any according to requirements without because of this moving outside the protection scope of the following claims.

## Claims

1. Supporting device (1) for containers (R) of biomedical fluids for parenteral administering, particularly bottles and phials for medical fluids, blood or the like, comprising at least one cup element (2) substantially shaped like a cup, having at least one upper fitting opening (3) for fitting a container for biomedical fluids and at least one lower exit opening (4) of the fluid, said cup element (2) comprising at least one outer surface (5) made of heat insulating material and at least one inner surface (6) suitable for facing said container (R),
**characterized in that** at least one portion of said inner surface (6) is associated with heat energy conduction and accumulation means (7, 8, 9),
wherein, during an initial phase with said supporting device (1) separate from said container (R), the heat energy conduction and accumulation means (7) are provided for accumulating heat energy, and
wherein afterwards, with the container fitted on the cup element (2) of said supporting device (1), the heat energy initially accumulated by said conduction and accumulation means (7) is progressively transferred to said container ( R), so as to maintain the temperature of said container (R) in the time, and
**in that** said conduction and accumulation means (7) comprise at least one first layer (8) made of heat conductive material.

2. Device (1) according to claim 1, **characterized in that** said conduction and accumulation means (7) comprise at least one second layer (9) made of heat insulating material substantially placed in between said outer surface (5) and said portion of inner surface (6).

3. Device according to claim 1, **characterized in that** said cup element (2) is rigid.

4. Device according to claim 1, **characterized in that** said heat insulating material of said outer surface !is substantially of the polymeric type.

5. Device according to claim 1, **characterized in that** the heat conductive material of said first layer (8) is of the metal type.

6. Device according to claim 5, **characterized in that** said heat conductive material of metal type is aluminum.

7. Device according to claim1, **characterized in that** said inner surface (6) is defined by said first layer (8).

8. Device according to claim 2, **characterized in that** the heat insulating material of said second layer (9) is in the fluid state.

9. Device according to claim 1, **characterized in that** said inner surface is substantially complementary to the shape of said container and suitable for being placed in contact with said container.

10. Device according to claim 1, **characterized in that** the dimensions of said cup element (2) are substantially larger than said container (R) and **in that** it comprises dimension adapting means suitable for transmitting heat between said inner surface (6) and said container (R).

11. Device according to claim 10, **characterized in that** said adapting means comprise at least one additional body, substantially tubular in shape, at least partially made of heat conductive material and having an outer side, substantially complementary to said inner surface (6) and which can be placed in contact with this, and an inner side, substantially complementary to said container and which can be placed in contact with this.

12. Device according to claim 1, **characterized in that** it comprises closing means (11) for closing said fitting opening (3).

13. Device according to claim 12, **characterized in that** said closing means are of the removable type.

14. Device according to claim 12, **characterized in that** said closing means (11) comprise at least one cover (12) associable with the edge of said fitting opening (3).

15. Device according to claim 14, **characterized in that** said cover (12) comprises a semi-shell for covering the portion of said container (R) protruding from said fitting opening (3).

16. Device according to claim 14, **characterized in that** said closing means (11) comprise sealing means (13) which can be placed in between said cup element (2) and said cover (12).

17. Device according to claim 16, **characterized in that** said sealing means comprise at least one ring-shaped seal associated with the edge of said fitting opening.

18. Device according to claim 15, **characterized in that** said closing means comprise hinging means for hinging said cover to said cup element.

19. Device according to one or more of the preceding claims, **characterized in that** it comprises sight inspection means (14) for inspecting the level of said biomedical fluid inside said container (R).

20. Device according to claim 19, as dependent on claim 14, **characterized in that** said sight inspection means (14) comprise at least one window (15) made of substantially transparent material which is obtained through a side of at least one of said cup element (2) and said cover (12).

21. Device according to claim 20, **characterized in that** said window (15) is elongated in a substantially vertical direction.

22. Device according to claim 20, **characterized in that** said sight inspection means (14) comprise at least one reflecting body (16) associated with said inner surface at the diametrically opposite part with respect to said window (15).

23. Device according to claim 1, **characterized in that** it further comprises heating means (17) of said conduction and accumulation means (7, 8, 9), wherein said heating means (17) are associated with said cup element (2).

24. Device according to claim 23, **characterized in that** said heating means (17) are substantially placed in between said first layer (8) and said second layer (9).

25. Device according to claim 23, **characterized in that** said heating means comprise at least one electric resistor (17) arranged in contact with the inner side of said first layer (8).

26. Device according to claim 25, **characterized in that** it comprises packing means (18) suitable for preventing any fluid from flowing inside said cup element (2).

27. Device according to claim 23, **characterized in that** it comprises at least one central processing and control unit (25) associated with said cup element (2) and with said heating means (17) and suitable for controlling the heating of said conduction and accumulation means (7, 8, 9).

28. Device according to claim 1, **characterized in that** it comprises gripping means (19, 20) associated with said cup element (2) and suitable for allowing their mounting on a vertical upright (A).

29. Device according to claim 28, **characterized in that** said gripping means (19, 20) are of the clamp type.

30. Device according to claim 1, **characterized in that** it comprises two of said cup elements (2) associated between them.

## Patentansprüche

1. Haltevorrichtung (1) für Behälter (R) von biomedizinischen Flüssigkeiten für die parenterale Verabreichung, insbesondere Flaschen und Fläschchen für medizinische Flüssigkeiten, Blut oder dergleichen, enthaltend mindestens ein Becherelement (2), das im Wesentlichen wie ein Becher geformt ist und mindestens eine obere Einsetzöffnung (3) zum Einsetzen eines Behälters für biomedizinische Flüssigkeiten und mindestens eine untere Austrittsöffnung (4) für die Flüssigkeit hat, welches Becherelement (2) mindestens eine äußere Oberfläche (5) aufweist, die aus wärmeisolierendem Material hergestellt ist, und mindestens eine innere Oberfläche (6), die dafür geeignet ist, dem Behälter (R) gegenüber zu liegen,
**dadurch gekennzeichnet, dass** mindestens ein Abschnitt der inneren Oberfläche (6) mit Wärmeenergieleitungs- und Sammeleinrichtungen (7, 8, 9) verbunden ist,
wobei während einer Anfangsphase, in der die Haltevorrichtung (1) von dem Behälter (R) getrennt ist, die Wärmeenergieleitungs- und Sammeleinrichtung (7) zum Sammeln von Wärmeenergie vorgesehen ist, und
wobei danach, wenn der Behälter in das Becherelement (2) der Haltevorrichtung (1) eingesetzt ist, die anfänglich von der Leitungs- und Sammeleinrichtung (7) gesammelte Energie progressiv auf den Behälter (R) übertragen wird, um so die Temperatur des Behälters (R) während der Zeit aufrechtzuerhalten, und
dadurch, dass die Leitungs- und Sammeleinrichtung (7) mindestens eine erste Schicht (8) aufweist, die aus wärmeleitendem Material hergestellt ist.

2. Vorrichtung (1) nach Anspruch 1, **dadurch gekennzeichnet,**
**dass** die Leitungs- und Sammeleinrichtung (7) mindestens eine zweite Schicht (9) aufweist, die aus wärmeisolierendem Material hergestellt ist und im Wesentlichen zwischen der äußeren Oberfläche (5) und dem Abschnitt der inneren Oberfläche (6) angeordnet ist.

3. Vorrichtung nach Anspruch 1, **dadurch gekennzeichnet, dass** das Becherelement (2) starr ist.

4. Vorrichtung nach Anspruch 1, **dadurch gekennzeichnet, dass** das wärmeisolierende Material der äußeren Oberfläche im Wesentlichen dem Polymertyp entspricht.

5. Vorrichtung nach Anspruch 1, **dadurch gekennzeichnet, dass** das wärmeleitende Material der ersten Schicht (8) dem Metalltyp entspricht.

6. Vorrichtung nach Anspruch 5, **dadurch gekennzeichnet, dass** das wärmeleitende Material des Metalltyps Aluminium ist.

7. Vorrichtung nach Anspruch 1, **dadurch gekennzeichnet, dass** die innere Oberfläche (6) durch die erste Schicht (8) gebildet ist.

8. Vorrichtung nach Anspruch 2, **dadurch gekennzeichnet, dass** das wärmeisolierende Material der zweiten Schicht (9) im Fluidzustand ist.

9. Vorrichtung nach Anspruch 1, **dadurch gekennzeichnet, dass** die innere Oberfläche mit der Form des Behälters im Wesentlichen komplementär ist und dafür geeignet ist, in Kontakt mit dem Behälter platziert zu werden.

10. Vorrichtung nach Anspruch 1, **dadurch gekennzeichnet, dass** die Abmessungen des Becherelements (2) wesentlich größer sind als des Behälters (R), und dadurch, dass es Einrichtungen zur Anpassung der Abmessungen aufweist, die für die Übertragung von Wärme zwischen der inneren Oberfläche (6) und dem Behälter (R) geeignet sind.

11. Vorrichtung nach Anspruch 10, **dadurch gekennzeichnet, dass** die Anpassungseinrichtung mindestens einen zusätzlichen Körper aufweist, der im Wesentlichen rohrförmig ist, zumindest teilweise aus wärmeleitendem Material hergestellt ist und eine äußere Seite hat, die im Wesentlichen mit der inneren Oberfläche (6) komplementär ist und die in Kontakt mit dieser angeordnet werden kann, sowie eine innere Seite, die im Wesentlichen mit dem Behälter komplementär ist und die in Kontakt mit diesem angeordnet werden kann.

12. Vorrichtung nach Anspruch 1, **dadurch gekennzeichnet, dass** sie eine Verschlusseinrichtung (11) zum Verschließen der Einsetzöffnung (3) aufweist.

13. Vorrichtung nach Anspruch 12, **dadurch gekennzeichnet, dass** die Verschlusseinrichtung dem abnehmbaren Typ entspricht.

14. Vorrichtung nach Anspruch 12, **dadurch gekennzeichnet, dass** die Verschlusseinrichtung (11) mindestens eine Abdeckung (12) aufweist, die mit dem Rand der Einsetzöffnung (3) verbindbar ist.

15. Vorrichtung nach Anspruch 14, **dadurch gekennzeichnet, dass** die Abdeckung (12) eine Halbschale zum Abdecken des aus der Einsetzöffnung (3) vorragenden Abschnitts des Behälters (R) umfasst.

16. Vorrichtung nach Anspruch 14, **dadurch gekennzeichnet, dass** die Verschlusseinrichtung (11) eine Dichtungseinrichtung (13) aufweist, die zwischen dem Becherelement (2) und der Abdeckung (12) angeordnet werden kann.

17. Vorrichtung nach Anspruch 16, **dadurch gekennzeichnet, dass** die Dichtungseinrichtung mindestens eine ringförmige Dichtung aufweist, die mit dem Rand der Einsetzöffnung verbunden ist.

18. Vorrichtung nach Anspruch 15, **dadurch gekennzeichnet, dass** die Verschlusseinrichtung eine Scharniereinrichtung zur Scharnierverbindung der Abdeckung mit dem Becherelement aufweist.

19. Vorrichtung nach einem oder mehreren der vorhergehenden Ansprüche, **dadurch gekennzeichnet, dass** sie eine Sichtprüfungseinrichtung (14) zur Prüfung des Niveaus der biomedizinischen Flüssigkeit innerhalb des Behälters (R) aufweist.

20. Vorrichtung nach Anspruch 19 in Abhängigkeit von Anspruch 14, **dadurch gekennzeichnet, dass** die Sichtprüfungseinrichtung (14) mindestens ein aus im Wesentlichen transparentem Material hergestelltes Fenster (15) aufweist, welches durch eine Seite mindestens eines des Becherelements (2) und der Abdeckung (12) vorgesehen ist.

21. Vorrichtung nach Anspruch 20, **dadurch gekennzeichnet, dass** das Fenster (15) in einer im Wesentlichen senkrechten Richtung länglich ist.

22. Vorrichtung nach Anspruch 20, **dadurch gekennzeichnet, dass** die Sichtprüfungseinrichtung (14) mindestens einen reflektierenden Körper (16) aufweist, der mit der inneren Oberfläche an einem in Bezug auf das Fenster (15) diametral gegenüberliegenden Teil verbunden ist.

23. Vorrichtung nach Anspruch 1, **dadurch gekennzeichnet, dass** sie ferner eine Heizeinrichtung (17) der Leitungs- und Sammeleinrichtung (7, 8, 9) enthält, wobei die Heizeinrichtung (17) mit dem Becherelement (2) verbunden ist.

24. Vorrichtung nach Anspruch 23, **dadurch gekennzeichnet, dass** die Heizeinrichtung (17) im Wesentlichen zwischen der ersten Schicht (8) und der zweiten Schicht (9) angeordnet ist.

25. Vorrichtung nach Anspruch 23, **dadurch gekennzeichnet, dass** die Heizeinrichtung mindestens einen elektrischen Widerstand (17) aufweist, der in Kontakt mit der inneren Seite der ersten Schicht (8) angeordnet ist.

26. Vorrichtung nach Anspruch 25, **dadurch gekennzeichnet, dass** sie eine Packungseinrichtung (18) aufweist, die dafür geeignet ist, zu verhindern, dass Flüssigkeit in das Innere des Becherelements (2) fließt.

27. Vorrichtung nach Anspruch 23, **dadurch gekennzeichnet, dass** sie mindestens eine Zentralverarbeitungs- und Regeleinheit (25) in Verbindung mit dem Becherelement (2) und mit der Heizeinrichtung (17) aufweist, die dafür geeignet ist, die Erwärmung der Leitungs- und Sammeleinrichtung (7, 8, 9) zu regeln.

28. Vorrichtung nach Anspruch 1, **dadurch gekennzeichnet, dass** sie eine mit dem Becherelement (2) verbundene Greifeinrichtung (19, 20) aufweist, welche dafür geeignet ist, ihre Anbringung an einem senkrechten Ständer (A) zu erlauben.

29. Vorrichtung nach Anspruch 28, **dadurch gekennzeichnet, dass** die Greifeinrichtung (19, 20) dem Klemmentyp entspricht.

30. Vorrichtung nach Anspruch 1, **dadurch gekennzeichnet, dass** sie zwei der Becherelemente (2) aufweist, die untereinander verbunden sind.

## Revendications

1. Dispositif de support (1) pour des contenants (R) de fluides biomédicaux destinés à une administration parentérale, en particulier des bouteilles et des flacons pour fluides médicaux, sang et analogues, comprenant au moins un élément cupulaire (2) essentiellement en forme de godet, ayant au moins une ouverture supérieure d'insertion (3) pour insérer un contenant destiné à des fluides biomédicaux et au moins une ouverture inférieure de sortie (4) du fluide, ledit élément cupulaire (2) comprenant au moins une surface extérieure (5) faite d'un matériau calorifuge et au moins une surface intérieure (6) apte à se trouver face audit contenant (R),
*caractérisé en ce qu*'au moins une portion de ladite surface intérieure (6) est associée à des moyens de conduction et d'accumulation d'énergie thermique (7, 8, 9),
dans lequel, pendant une phase initiale où ledit dispositif de support (1) est séparé dudit contenant (R), les moyens de conduction et d'accumulation d'énergie thermique (7) sont prévus pour accumuler l'énergie thermique, et
dans lequel, ensuite, lorsque le contenant est inséré dans l'élément cupulaire (2) dudit dispositif de support (1), l'énergie thermique initialement accumulée par lesdits moyens de conduction et d'accumulation (7) est progressivement transférée audit contenant (R) de façon à maintenir dans le temps la température dudit contenant (R), et
*en ce que* lesdits moyens de conduction et d'accumulation (7) comprennent au moins une première couche (8) faite d'un matériau thermo-conducteur.

2. Dispositif (1) selon la revendication 1, ***caractérisé en ce que*** lesdits moyens de conduction et d'accumulation (7) comprennent au moins une deuxième couche (9) faite d'un matériau calorifuge essentiellement placé entre ladite surface extérieure (5) et ladite portion de surface intérieure (6).

3. Dispositif selon la revendication 1, ***caractérisé en ce que*** ledit élément cupulaire (2) est rigide.

4. Dispositif selon la revendication 1, ***caractérisé en ce que*** ledit matériau calorifuge de ladite surface extérieure est essentiellement du type polymère.

5. Dispositif selon la revendication 1, ***caractérisé en ce que*** ledit matériau thermo-conducteur de ladite première couche (8) est du type métallique.

6. Dispositif selon la revendication 5, ***caractérisé en ce que*** ledit matériau thermo-conducteur de type métallique est l'aluminium.

7. Dispositif selon la revendication 1, ***caractérisé en ce que*** ladite surface intérieure (6) est définie par ladite première couche (8).

8. Dispositif selon la revendication 2, ***caractérisé en ce que*** ledit matériau calorifuge de ladite deuxième couche (9) est à l'état fluide.

9. Dispositif selon la revendication 1, ***caractérisé en ce que*** ladite surface intérieure est essentiellement complémentaire de la forme dudit contenant et apte à être mise en contact avec ledit contenant.

10. Dispositif selon la revendication 1, ***caractérisé en ce que*** les dimensions dudit élément cupulaire (2) sont sensiblement supérieures à celles dudit contenant (R) et *en ce qu*'il comprend des moyens d'adaptation des dimensions aptes à transmettre la chaleur entre ladite surface intérieure (6) et ledit contenant (R).

11. Dispositif selon la revendication 10, ***caractérisé en ce que*** lesdits moyens d'adaptation comprennent au moins un corps supplémentaire, de forme sensiblement tubulaire, au moins partiellement fait d'un matériau thermo-conducteur et ayant une face extérieure essentiellement complémentaire de ladite surface intérieure (6) et qui peut être placée en contact avec celle-ci, et une face intérieure essentiellement complémentaire dudit contenant et qui peut être placée en contact avec celui-ci.

12. Dispositif selon la revendication 1, ***caractérisé en ce* qu'**il comprend des moyens de fermeture (11) pour refermer ladite ouverture d'insertion (3).

13. Dispositif selon la revendication 12, ***caractérisé en ce que*** lesdits moyens de fermeture sont du type amovible.

14. Dispositif selon la revendication 12, ***caractérisé en ce que*** lesdits moyens de fermeture (11) comprennent au moins un couvercle (12) associable au rebord de ladite ouverture d'insertion (3).

15. Dispositif selon la revendication 14, ***caractérisé en ce que*** ledit couvercle (12) comprend une demi-coque pour recouvrir la portion dudit contenant (R) faisant saillie de ladite ouverture d'insertion (3).

16. Dispositif selon la revendication 14, ***caractérisé en ce que*** lesdits moyens de fermeture (11) comprennent des moyens d'étanchéité (13) qui peuvent être placés entre ledit élément cupulaire (2) et ledit couvercle (12).

17. Dispositif selon la revendication 16, ***caractérisé en ce que*** lesdits moyens d'étanchéité comprennent au moins un joint torique associé au rebord de ladite ouverture d'insertion.

18. Dispositif selon la revendication 15, ***caractérisé en ce que*** lesdits moyens de fermeture comprennent des moyens de charnière pour articuler ledit couvercle audit élément cupulaire.

19. Dispositif selon l'une ou plusieurs des revendications précédentes, *caractérisé en ce qu*'il comprend des moyens d'inspection visuelle (14) pour inspecter le niveau dudit fluide biomédical à l'intérieur dudit contenant (R).

20. Dispositif selon la revendication 19 lorsqu'elle est dépendante de la revendication *14, **caractérisé en ce que*** lesdits moyens d'inspection visuelle (14) comprennent au moins une fenêtre (15) en matériau essentiellement transparent, qui est ménagée à travers une face d'au moins l'un d'entre ledit élément cupulaire (2) et ledit couvercle (12).

21. Dispositif selon la revendication 20, ***caractérisé en ce que*** ladite fenêtre (15) est allongée dans une direction essentiellement verticale.

22. Dispositif selon la revendication 20, ***caractérisé en ce que*** lesdits moyens d'inspection visuelle (14) comprennent au moins un corps réfléchissant (16) associé à ladite surface intérieure, situé au niveau de la partie diamétralement opposée à ladite fenêtre (15).

23. Dispositif selon la revendication 1, *caractérisé en ce qu*'il comprend en outre des moyens de chauffage (17) desdits moyens de conduction et d'accumulation (7, 8, 9), dans lequel lesdits moyens de chauffage (17) sont associés audit élément cupulaire (2).

24. Dispositif selon la revendication 23, ***caractérisé en ce que*** lesdits moyens de chauffage (17) sont essentiellement placés entre ladite première couche (8) et ladite deuxième couche (9).

25. Dispositif selon la revendication 23, ***caractérisé en ce que*** lesdits moyens de chauffage comprennent au moins une résistance électrique (17) placée en contact avec la face intérieure de ladite première couche (8).

26. Dispositif selon la revendication 25, ***caractérisé en ce qu'**il* comprend des moyens de garniture (18) aptes à empêcher tout fluide de s'écouler à l'intérieur dudit élément cupulaire (2).

27. Dispositif selon la revendication 23, ***caractérisé en ce qu'**il* comprend au moins une unité centrale de traitement et de contrôle (25) associée audit élément cupulaire (2) et auxdits moyens de chauffage (17) et apte à contrôler le chauffage desdits moyens de conduction et d'accumulation (7, 8, 9).

28. Dispositif selon la revendication 1, ***caractérisé en ce qu'**il* comprend des moyens de serrage (19, 20) associés audit élément cupulaire (2) et aptes à permettre leur montage sur un montant vertical (A).

29. Dispositif selon la revendication 28, ***caractérisé en ce que*** lesdits moyens de serrage (19, 20) sont du type étau.

30. Dispositif selon la revendication 1, ***caractérisé en ce qu'**il* comprend deux desdits éléments cupulaires (2) associés entre eux.
